# EUROPEAN PATENT APPLICATION

(11) **EP 2 514 455 A1**
(43) Date of publication of application: **24.10.2012**
(21) Application number: 11163409.3
(22) Date of filing: 21.04.2011
(51) Int. Cl.: A61M 5/24, B65D 83/02, B65D 85/24, A61M 5/315, A61M 5/00, A61J 1/14, A61J 1/16, A61J 1/20

(54) **Injection pen with revolver containing medicated modules**

(71) Applicant: Sanofi-Aventis Deutschland GmbH, 65929 Frankfurt am Main (DE)
(72) Inventor: Young, Alasdair George, OX7 5ER, Chipping Norton Oxfordshire (GB); Mercer, David Richard, Warwickshire CV31 3BY (GB); De Sausmarez Lintell, Daniel Thomas, Rugby, Warwickshire CV23 9EQ (GB)

(57) **Abstract**

A cassette (4) of medicated modules for an injection system to co-deliver at least two medicaments is disclosed where a primary delivery device (7) containing a primary medicament accepts the cassette containing two or more medicated module each containing a single dose of a secondary medicament and where both medicaments are delivered through a hollow needle (3).

## Description

### Field of the Present Patent Application

This invention relates to medical devices and methods of delivering at least two drug agents from separate reservoirs using devices having only a single dose setting mechanism and a single dispense interface. A single delivery procedure initiated by the user causes a non-user settable dose of a second drug agent and a variable set dose of a first drug agent to be delivered to the patient. The drug agents may be available in two or more reservoirs, containers or packages, each containing independent (single drug compound) or pre-mixed (co-formulated multiple drug compounds) drug agents. Specifically, our invention concerns a cassette, attachable to a primary device, that contains two or more medicated modules or reservoirs each containing a fixed single dose of a second drug agent, where the cassette is rotated to expose a fresh module of medicament for each use. Our invention is of particular benefit where the therapeutic response can be optimized for a specific target patient group, through control and definition of the therapeutic profile.

### Background

Certain disease states require treatment using one or more different medicaments. Some drug compounds need to be delivered in a specific relationship with each other in order to deliver the optimum therapeutic dose. This invention is of particular benefit where combination therapy is desirable, but not possible in a single formulation for reasons such as, but not limited to, stability, compromised therapeutic performance and toxicology.

For example, in some cases it might be beneficial to treat a diabetic with a long acting insulin and with a glucagon-like peptide-1 (GLP-1), which is derived from the transcription product of the proglucagon gene. GLP-1 is found in the body and is secreted by the intestinal L cell as a gut hormone. GLP-1 possesses several physiological properties that make it (and its analogs) a subject of intensive investigation as a potential treatment of diabetes mellitus.

There are a number of potential problems when delivering two medicaments or active agents simultaneously. The two active agents may interact with each other during the long-term, shelf life storage of the formulation. Therefore, it is advantageous to store the active components separately and only combine them at the point of delivery, e.g. injection, needle-less injection, pumps, or inhalation. However, the process for combining the two agents needs to be simple and convenient for the user to perform reliably, repeatedly and safely.

A further problem is that the quantities and/or proportions of each active agent making up the combination therapy may need to be varied for each user or at different stages of their therapy. For example one or more actives may require a titration period to gradually introduce a patient up to a "maintenance" dose. A further example would be if one active requires a non-adjustable fixed dose while the other is varied in response to a patient's symptoms or physical condition. This problem means that pre-mixed formulations of multiple active agents may not be suitable as these pre-mixed formulations would have a fixed ratio of the active components, which could not be varied by the healthcare professional or user.

Additional problems arise where a multi-drug compound therapy is required, because many users cannot cope with having to use more than one drug delivery system or make the necessary accurate calculation of the required dose combination. This is especially true for users with dexterity or computational difficulties. In some circumstances it is also necessary to perform a priming procedure of the device and/or needle cannulae before dispensing the medicaments. Likewise, in some situations, it may be necessary to bypass one drug compound and to dispense only a single medicament from a separate reservoir.

Accordingly, there exists a strong need to provide devices and methods for the delivery of two or more medicaments in a single injection or delivery step that is simple for the user to perform. Our invention overcomes the above-mentioned problems by providing separate storage containers for two or more active drug agents that are then only combined and/or delivered to the patient during a single delivery procedure. Setting a dose of one medicament automatically fixes or determines the dose of the second medicament (i.e. non-user settable). Our invention also gives the opportunity for varying the quantity of one or both medicaments. For example, one fluid quantity can be varied by changing the properties of the injection device (e.g. dialing a user variable dose or changing the device's "fixed" dose). The second fluid quantity can be changed by manufacturing a variety of secondary drug containing packages with each variant containing a different volume and/or concentration of the second active agent. The user or healthcare professional would then select the most appropriate secondary package or series or combination of series of different packages for a particular treatment regime.

Our invention also provides a cassette containing a number of individual modules or reservoirs, each comprising a single, fixed dose of a secondary medicament, where rotation of the cassette housing automatically causes the reservoir to come into fluid communication with the primary medicament. This allows the user to perform repeated administrations of a secondary medicament using a single cassette.

These and other advantages will become evident from the following more detailed description of the invention.

### SUMMARY

Our invention allows complex combinations of multiple drug compounds within a single drug delivery system. The invention allows the user to set and dispense a multi-drug compound device though one single dose setting mechanism and a single dispense interface. This single dose setter controls the mechanism of the device such that a predefined combination of the individual drug compound is delivered when a single dose of one of the medicaments is set and dispensed through the single dispense interface.

By defining the therapeutic relationship between the individual drug compounds our delivery device would help ensure that a patient/user receives the optimum therapeutic combination dose from a multi-drug compound device without the inherent risks associated with multiple inputs where the user has to calculate and set the correct dose combination every time they use the device. The medicaments can be fluids, defined herein as liquids or powders that are capable of flowing and that change shape at a steady rate when acted upon by a force tending to change its shape. Alternatively, one of the medicaments may be a solid that is carried, solubilized or otherwise dispensed with another fluid medicament.

According to one specific aspect, this invention is of particular benefit to users with dexterity or computational difficulties as the single input and associated predefined therapeutic profile removes the need for them to calculate their prescribed dose every time they use the device and the single input allows considerably easier setting and dispensing of the combined compounds.

In a preferred embodiment a master or primary drug compound, such as insulin, contained within a multiple dose, user selectable device could be used with a single use, user replaceable, module that contains a single dose of a secondary medicament and the single dispense interface. When connected to the primary device the secondary compound is activated/delivered on dispense of the primary compound. Although our invention specifically mentions insulin, insulin analogs or insulin derivatives, and GLP-1 or GLP-1 analogs as two possible drug combinations, other drugs or drug combinations, such as an analgesics, hormones, beta agonists or corticosteroids, or a combination of any of the above-mentioned drugs could be used with our invention.

For the purposes of our invention the term "insulin" shall mean Insulin, insulin analogs, insulin derivatives or mixtures thereof, including human insulin or a human insulin analogs or derivatives. Examples of insulin analogs are, without limitation, Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin or Des(B30) human insulin. Examples of insulin derivatives are, without limitation, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

As used herein the term "GLP-1" shall mean GLP-1, GLP-1 analogs, or mixtures thereof, including without limitation, exenatide (Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2), Exendin-3, Liraglutide, or AVE0010 (H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Ser-Lys-Lys-Lys-Lys-Lys-Lys-NH2).

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

In one embodiment of our invention there is provided a cassette of medicated modules attachable to a drug delivery device that comprises an outer housing and an inner housing having a proximal end having an upper hub holding a first double-ended needle cannula configured for attachment to a primary drug delivery device. The cassette has a primary pack chassis comprising two or more reservoirs each containing a single, fixed dose of a medicament. The chassis further comprises a number of cages that are configured to hold the reservoirs. Each cage has an outside surface that has a connector to engage a removable dispense interface, preferably needle hub, where the connector is most preferably a threaded connection.

At least one of these reservoirs is configured as a priming module to allow the cassette to be primed before use. Priming of the cassette is a procedure to remove any entrained or trapped air within the flow paths, particularly within the needle cannula. The priming module is preferably the first module in position or indexed in the cassette and does not contain a reservoir of the secondary medicament. Instead, the priming module is straight conduit of minimal volume that connects the upper needle cannula with the lower needle cannula and operates as a pass through to allow a small quantity of primary medicament to be passed through (dispensed) in order to ensure the upper needle is liquid full when the first medicated module is indexed into position. This also ensures dose accuracy of the primary medicament. Before the first medicated module is indexed into position, the user would set a small dose of primary medicament using the primary dose delivery device and would dispense this set dose through the priming module within the attached cassette and thus prime the cassette.

A cut-out at the distal end of the outer housing is configured such that one cage at a time is accessible for installing a dispense interface, preferably a double ended needle cannula mounted in a hub. The cut-out is sized to prevent the cassette from being rotated when a needle hub is connected to a cage connector. The outer housing has an outer surface containing one or more gripping surfaces that allows the user to rotate the outer housing relative to the inner housing causing the chassis to rotate and to expose a cage containing a full reservoir for attachment to the upper needle and to a removable dispense interface. Attachment of the dispense interface triggers an indicator covering mechanism that partially obscures, blocks or shields an indicator that is normally visible in a window in the outer housing.

The indicator visible through a window in the distal portion of the outer housing is part of a decrementing mechanism that keeps track of the state of the cassette as it is used. For example, if the cassette holds five medicated modules and one priming module, the decrementing mechanism might have an indicator that the shows a "P" for the priming module being in an active position and the numbers "5" through "1" for each of the medicated modules that are sequentially placed in an active position. The indicator can also be a pip, knob, button, or the like that protrudes through the window in outer housing and tactilely informs the user whether the module is in the pre-use or ready-to-use state. It may also be a visual indicator, e.g. showing colors or symbols, or a tactile or audible indicator. Preferably, user noticeable indicia indicate a pre-use priming position, module number, and an end of life locked position of the cassette when all medicated modules have been used. The cassette may further also comprise means that are configured to prevent further rotation of the outer housing when all medicated modules have been used thereby preventing reuse of the cassette.

Activation of indicator cover is non-reversible, even after removal of the hub of the dispense interface. This provides the user with a visual sign that the module has been used and that the cassette must be activated to position a new module in the cut-out. Likewise, the cover on the indicator may indicate to the user to remove and dispose of the used dispense interface. The cassette can also have an end of use lockout that would prevent the outer housing and chassis from rotating to place another module in an active position for engagement of the upper needle. In operation the user would rotate the outer housing in a first direction to deselect a used module and to withdraw the upper needle from engagement with that used module. Rotation in the first direction causes the inner housing and outer housing to move apart from each other along a helical path. This axial separation of the two housings allows the upper needle to withdraw from engagement with either the one priming module or one of the medicated modules. After rotating in the first direction, rotation of outer housing in a second direction will index the cassette so that a new module will engage the upper needle and a new cage will be exposed in the cut-out to attach a dispense interface. A biasing element can be used between the outer and inner housings as a return mechanism during rotation in the first direction. This biasing element is preferably a compression spring.

Although a number of dispense interfaces, such as a Type A needle, can be used with the cassette modules, it is preferred that when a needle is used as the dispense interface that needle assembly contains a safety guard to reduce the risk of accidental needle sticks before and after use, reduce the anxiety of users suffering from needle phobia as well as preventing a user from using the device a subsequent time. The needle guard is preferably configured with a solid planar surface at its distal end that provides a large surface area that reduces the pressure exerted on the patient's skin, which allows the user to experience an apparent reduction in the force exerted against the skin. Preferably, the planar surface covers the entire distal end of the guard with the exception of a small needle pass through hole aligned axially with the needle. This pass through hole is preferably no more than 10 times greater in diameter than the outer diameter of the needle cannula. For example, with a needle outside diameter of 0.34mm, the pass through hole diameter can be from about 3 to about 4mm. Preferably, the pass through hole size should be large enough for the user to see that the device is primed (i.e., a drop or more of medicament) while not being so large that it is still possible to reach the end of the needle with a finger (i.e. needle stick injuries before or after use). This difference between the hole size and cannula diameter is to allow for tolerances, to allow users to see the drop of liquid on the end of the cannula after priming (whether a transparent or non-transparent guard is used) while keeping the size small enough to prevent accidental needle stick injuries.

Further, the needle guard or shield is configured to move axially in both the distal and proximal directions when pressed against and removed from an injection site. When the needle assembly is removed or withdrawn from the patient, the guard is returned to post-use extended position. A locking mechanism on the guard, rotating cylinder, or combination of both can be used to securely lock the guard from further substantial axial movement at the completion of the injection. By "substantial" movement we do not mean the typical amount of "play" in a system, but instead we mean that the guard and/or distal needle do not move axially a distance that exposes the distal end of the cannula once it is locked out.

In one embodiment of our invention there is provided a cassette of medicated modules as described herein attachable to a drug delivery device, preferably a pen shaped injection device, where the needle assembly comprises an upper hub holding a first double-ended needle cannula and a connector configured for attachment to a drug delivery device. The hub can be a separate part from the housing or integral, for example molded as part of the housing. The connector can be any connector design, such as threads, snap fits, bayonet, luer lock, or combination of these designs.

Preferably, a double-ended needle cannula is used for the upper or proximal cannula piercing a septum or seal in both the module reservoir and the cartridge of primary medicament in the injection device. The proximal needle is mounted in the upper hub of the outer housing, each using any technique known to those skilled in the art, such as welding, gluing, friction fit, over-molding and the like.

During dispense, substantially the entire amount of second medicament has been expelled as well as the selected or dialed dose of the first medicament, through the single dispense interface. The reservoir preferably contains a flow distributor to ensure that substantially all the single dose of secondary medicament is forced out of the capsule by the primary medicament during an injection. The flow distributor can be a separate stand-alone insert or pin. Alternatively, the flow distributor and the capsule together can be manufactured or assembled as a one-piece component where the flow distributor is integral with the reservoir or capsule. Such a unitary construction can be achieved utilizing , for example, design principles such as form fit, force fit or material fit, such as welding, gluing, or the like, or any combination thereof. The one-piece component may comprise one or more medicament flow channels, preferably one flow channel. The capsule and/or flow distributor can be constructed of any material that is compatible to the primary and secondary medicaments. Preferably the capsule and/or flow distributor can be made from compatible materials of construction that include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). Further preferred materials include those that are typically used to manufacture septa or pistons (bungs) found in multi-dose medicament cartridges, however, any other material that is compatible with the drug could be used, e.g., glass, plastics or specific polymers, for example, TPE (thermo plastic elastomer); LSR (liquid silicone rubber); LDPE (low density polyethylene); and/or any kind of medical grade rubber, natural or synthetic.

By "substantially all" we mean that at least about 80% of the second medicament is expelled from the drug delivery device, preferably at least about 90% is expelled. In the third state, preferably the module is locked so as to prevent a second delivery or insertion by means of a locking mechanism.

The combination of compounds as discrete units or as a mixed unit is delivered to the body via an integral needle. This would provide a combination drug injection system that, from a user's perspective, would be achieved in a manner that very closely matches the currently available injection devices that use standard needles.

The cassette of the invention can be designed for use with any drug delivery device with an appropriate compatible interface. However, it may be preferable to design the cassette in such a way as to limit its use to one exclusive primary drug delivery device (or family of devices) through employment of dedicated/coded/exclusive features to prevent attachment of a non-appropriate cassette to a non-matching device. In some situations it may be beneficial to ensure that the cassette is exclusive to one drug delivery device while also permitting the attachment of a standard drug dispense interface to the device. This would allow the user to deliver a combined therapy when the cassette is attached, but would also allow delivery of the primary compound independently through a standard drug dispense interface in situations, such as, but not limited to, dose splitting or top-up of the primary compound.

A particular benefit of our invention is that the cassette makes it possible to tailor dose regimes when required, especially where a titration period is necessary for a particular drug. The medicated modules within the cassette could be supplied in a number of titration levels with obvious differentiation features such as, but not limited to, aesthetic design of features or graphics, numbering etc, so that a patient could be instructed to use the supplied medicated module in a specific order to facilitate titration. Alternatively, the prescribing physician may provide the patient with a number of "level one" titration medicated modules and then when these were finished, the physician could then prescribe the next level. A key advantage of this titration program is that the primary device remains constant throughout.

In a preferred embodiment of our invention, the primary drug delivery device is used more than once and therefore is multi-use; however, the drug delivery device may also be a single use disposable device. Such a device may or may not have a replaceable reservoir of the primary drug compound, but our invention is equally applicable to both scenarios. It is also possible to have a suite of different medicated modules or cassettes for various conditions that could be prescribed as one-off extra medication to patients already using a standard drug delivery device.

A further feature of our invention is that both medicaments are delivered via one injection needle and in one injection step. This offers a convenient benefit to the user in terms of reduced user steps compared to administering two separate injections. This convenience benefit may also result in improved compliance with the prescribed therapy, particularly for users who find injections unpleasant or who have computational or dexterity difficulties.

Our invention also covers a method of delivering two medicaments stored in separate primary packages. The medicaments may both be liquid, or alternatively one or more of the medicaments may be a powder, suspension or slurry. In one embodiment the medicated module could be filled with a powdered medicament that is either dissolved or entrained in the primary medicament as it is injected through the medicated module.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates one possible system where one embodiment of the cassette of this invention is combined with a primary drug delivery device;
Figure 2 illustrates the combination of the cassette and drug delivery device shown in Fig. 1 with an attached Type A needle;
Figure 3 illustrates a perspective close-up view of the cut-out portion of the cassette illustrated in Fig. 1;
Figure 4 illustrates a transparent perspective view of the cassette illustrated in Figure 1;
Figure 5 is a side view of the embodiment of Figure 1;
Figure 6 illustrates a cross-sectional view of the cassette illustrated in Figure 1 when the outer and inner housing are extended and the upper needle is not engaged with a module;
Figure 7 illustrates a cross-sectional view of the cassette illustrated in Figure 1 when the outer and inner housing are moved together and the upper needle is engaged with a module; and
Figure 8 illustrates a perspective view and a sectional view of the priming module of the cassette illustrated in Figure 1.

### DETAILED DESCRIPTION

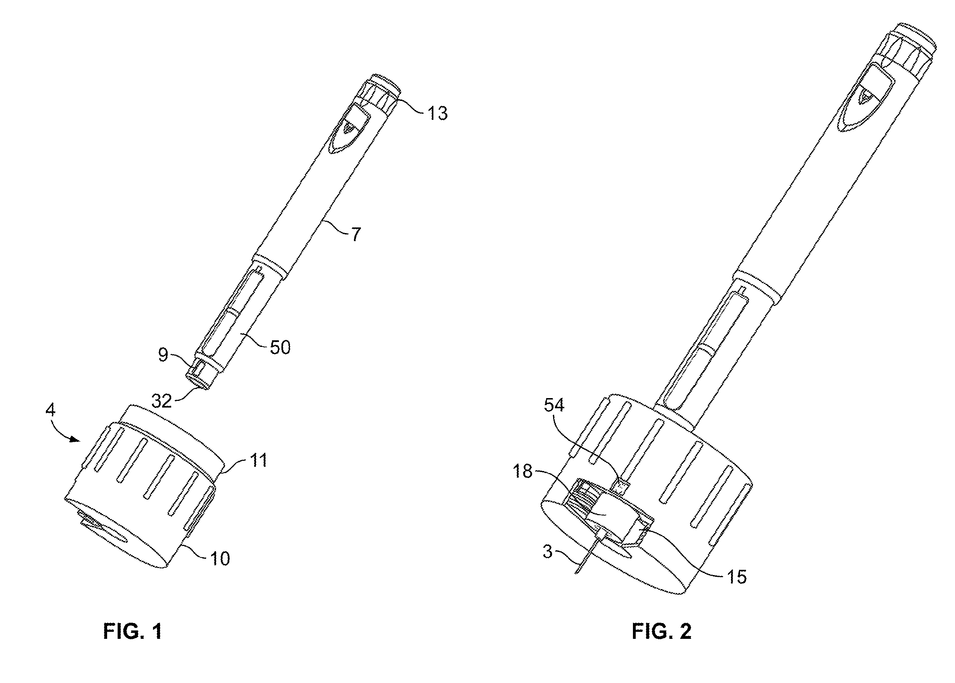

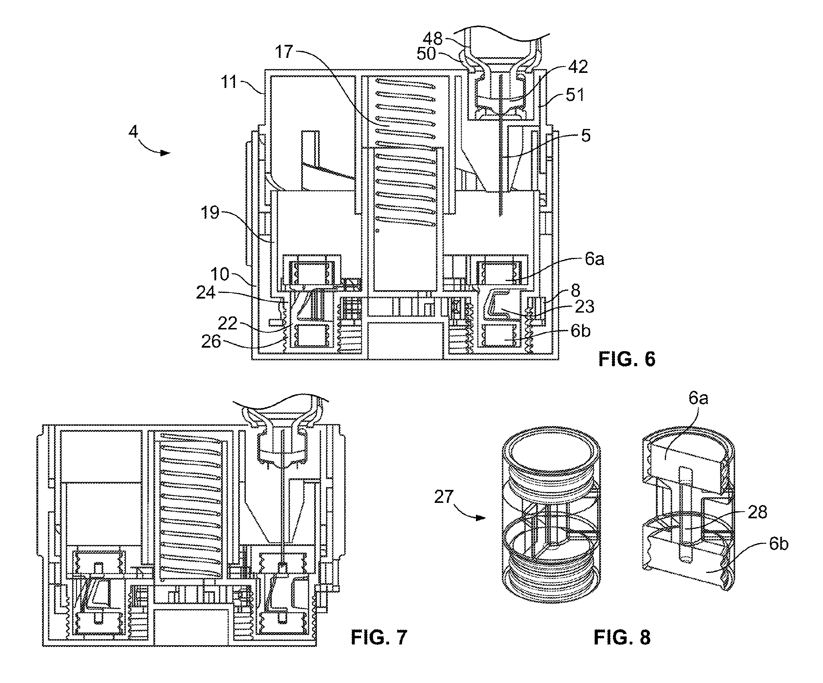

The cassette of the invention preferably holds five medicated modules and one priming module, such that when connected to a primary drug delivery device 7 each medicated module can administer a fixed predetermined dose of a secondary drug compound (medicament) and a variable dose of a primary or first drug compound through a single output or drug dispense interface, preferably a double ended needle cannula mounted in a hub (18). Setting the dose of the primary medicament by the user automatically determines the fixed dose of the second medicament, which preferably is a single dose contained in a capsule or reservoir in each module and that has an integral flow distributor. Fig. 1 illustrates one example of a system with a drug delivery device 7 and a cassette 4 of our invention that can be attached to the connection means 9 on cartridge holder 50 of distal end 32. Each cassette is preferably self-contained and provided as a sealed and sterile disposable assembly that has an attachment means in an upper hub 51 (see Fig. 6) that is compatible to the attachment means 9 at the distal end 32 of the drug delivery device 7. Although not shown, the cassette 4 could be supplied by a manufacturer in a protective and sterile container, where the user would peel or rip open a seal or the container itself to gain access to the sterile cassette. In some instances it might be desirable to provide two or more seals for each end of the cassette.

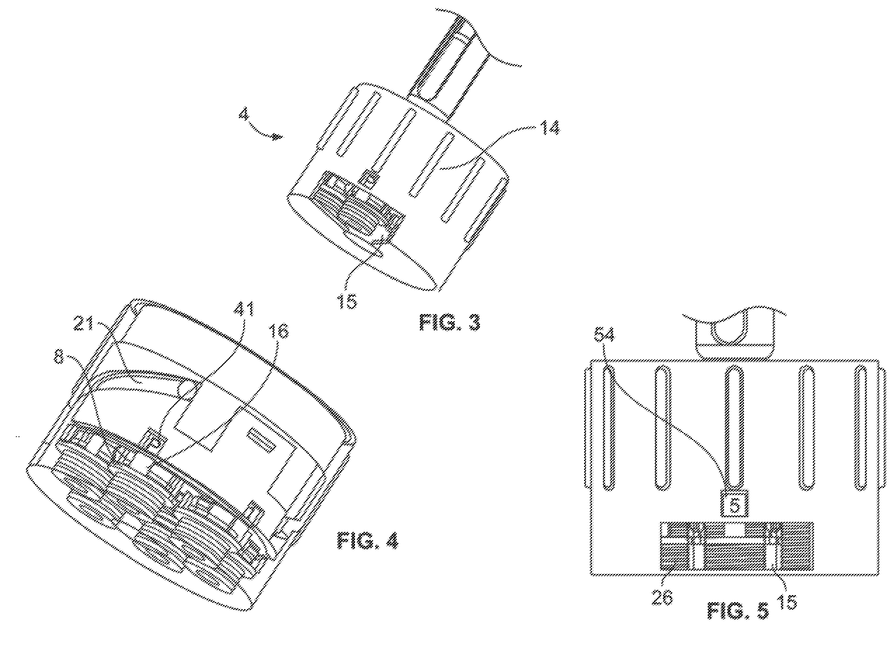

Any known attachment means can be used to attach the cassette to the chosen drug delivery device, including all types of permanent and removable connection means, such as threads, snap locks, snap fits, luer locks, bayonet, snap rings, keyed slots, and combinations of such connections. Fig. 1 illustrates the attachment means 9 as a unique connection that is keyed specifically to a corresponding connection on cassette 4. The embodiment shown in Fig. 4 illustrates a total of six modules within the cassette where five of the modules contain a single dose of a secondary medicament being contained entirely within reservoir 22, hence minimizing the risk of material incompatibility between the second medicament and the materials used in the construction of the cassette 4, specifically outer housing 10, inner housing 11, or any of the other parts used in the construction of the cassette.

To minimize the residual volume of the second medicament, caused by recirculation and/or stagnant zones, that might remain in reservoir 22 at the end of the dispense operation, it is preferable to have a flow distributor 23 as an integral part of reservoir 22 (see Fig. 6). The reservoir 22 containing the single dose of the secondary medicament can be sealed with septa 6a and 6b. Fig. 8 shows the priming module 27 with priming channel 28 that provides a fluid path to allow priming of the drug delivery device before the first injection using one of medicated modules in the cassette. Preferably the reservoirs, priming channel and flow distributors can be made from materials that are compatible with the primary medicament. Examples of compatible materials of construction include, but are not limited to, COC (an amorphous polymer based on ethylene and norbonene, also referred to as cyclic olefin copolymer, ethylene copolymer, cyclic olefin polymer, or ethylene-norbornene copolymer); LCP (a liquid crystal polymer having an aramid chemical structure that includes linearly substituted aromatic rings linked by amide groups, and further can include partially crystalline aromatic polyesters based on p-hydroxybenzoic acid and related monomers and also highly aromatic polyesters); PBT (polybutylene terephthalate thermoplastic crystalline polymer or polyester); COP (a cyclic olefin polymer based on ring-opening polymerization of norbornene or norbornene-derivatives); HDPE (high density polyethylene); and SMMA (styrene methyl methacrylate copolymer based on methyl methacrylate and styrene). The needle pierceable septa, bungs, and/or seals that are used with both the reservoir and the primary medicament cartridge can be manufactured using TPE (thermo plastic elastomer); LSR (liquid silicone rubber); LDPE (low density polyethylene); and/or any kind of medical grade rubber, natural or synthetic.

The design of flow distributor 23 should ensure that at least about 80% of the second medicament is expelled from reservoir 22 through the distal end of needle 3. Most preferably at least about 90% should be expelled. Ideally, displacement of the first medicament in a primary reservoir (not shown) contained in cartridge holder 50 and through the reservoir will displace the single dose of the second medicament stored in reservoir 22 without substantial mixing of the two medicaments.

Attachment of the cassette 4 to the multi-use device 7 causes an upper or proximal needle 5 mounted in upper hub 51 of inner housing 11 to penetrate a septum 42 sealing the distal end of cartridge 48 of primary medicament positioned in cartridge holder 50 of the multi-use device 7. Once the needle in upper hub has passed through the septum of the cartridge, a fluid connection is made between the first medicament and the needle. At this point, the outer housing 10 and the inner housing 11 are spaced apart as illustrated in Fig. 6. To prime the system the user rotates the outer housing 10 in a second direction to bring the housings together as shown in Fig. 7 and to cause the needle 5 to engage the priming module 27 such that the needle comes into fluid communication with priming channel 28. Once a dispense interface 18, preferably a double ended needle, is attached to cage 24 that holds the priming module 27, then needle 3 likewise comes into fluid communication with the priming channel 28. A small dose of primary medicament is set using device 7 and delivered by activating button 13.

Once the device 7 is primed, the dispense interface 18 must be removed. Then, the outer housing 10 is rotated in a first direction to disengage needle 5 from the priming module. This is possible because rotation of the two housing along a helical path 21 (see Fig. 4) causes the outer and inner housing to move axially apart or away from each other. The first medicated module or reservoir 22 is then moved into position and becomes engaged with needle 5 by rotating the outer housing and chassis 19 in the second direction. A dispense interface 18, is then attached to cage 24 that holds the first medicated module or reservoir 22. At this point a dose of the primary medicament in device 7 can be set and delivered by activating dose button 13. The dose button of our invention can be any triggering mechanism that causes the dose of the first medicament that was set to move towards the distal end 32 of device 7 and flow through the reservoir 22 of cassette 4 and out needle 3. In a preferred embodiment the dose button is operably connected to a spindle that engages a piston in the primary reservoir of the first medicament. In a further embodiment the spindle is a rotatable piston rod comprising two distinct threads.

One embodiment of the cassette assembly 4 of the invention is illustrated in Figs. 1- 7. In these embodiments the five reservoirs 22 each contain a fixed single dose of a secondary medicament sealed between two seals 6a and 6b. In some cases this secondary medicament may be a mixture of two or more drug agents that can be the same or different from the primary drug compound in the drug delivery device 7. Preferably the reservoirs are permanently fixed within the cassette, however, in some cases it may be preferred to design the cassette such that individual modules can be removed when empty and replaced with a new modules.

As best viewed in Fig. 6, the outer housing 10 is connected to chassis 19 that has six cages 24 to hold the five medicated module and one priming module. Each reservoir 22 is contained in a cage that has an outside surface with connector 26 shown as threads in the drawings. The biasing element 17 is shown as a return spring and assists the user in rotating the outer housing 10 and chassis 19 in the first direction to disengage a module from needle 5 held in upper hub 51 of inner housing 11. A cover lock mechanism 8 is connected to chassis 19 and is associated with each outer surface of cages 24. A cover 16 (see Fig. 4) is associated with the cover lock 8 such that when a needle hub 18 is connected to the outer surface of cage 24, the cover lock 8 and cover 16 are pushed proximally to block indicator 41 (see Fig. 2). Indicator 41 is seen through a window 54 in the distal end of outer housing 10. The cover lock 8 and cover 16 remain in this blocking or covering position even after the needle hub 18 is removed. This provides the user with a visual reminder that the module associated with that particular cage has been used and that the cassette needs to be indexed to the next module.

The indicator 41 is part of a decrementing mechanism that essentially counts down the remaining unused modules in the cassette until all have been used. An end of use lock out mechanism (not shown) can be included in the cassette that prevents further rotation of the outer housing and chassis in one or both the first or second directions. Such a lock out mechanism could be one or more snap lock features that engages and locks the inner and housings 11 and 10, respectively, when the final module is indexed into position that prevents the user from rotating outer housing 10 in the opposite direction. Preferably the indicator has a separate visual symbol or letter for the priming module and numbers for each of the medicated modules or reservoirs 22. The cover 16 can be any visual indicator that partially or fully blocks viewing of indicator 41. To assist the user in rotating the outer housing in the first and second directions, grip surfaces 14 are spaced around the outside surface of outer housing 10. A cut-out 15 in the distal end of the outer housing 10 provides access to one cage connector 26 at a time and is configured such that the outer housing cannot be rotated when a needle hub or other dispense interface is attached to the cage.

In any of the above described embodiments of our invention the second medicament may be either in a powdered solid state, any fluid state contained within the reservoir, or coated to the inside surface of the drug dispense interface. The greater concentration of the solid form of the medicament has the benefit of occupying a smaller volume than the liquid having lower concentration. This in turn reduces the ullage of the medicated module reservoir. An additional benefit is that the solid form of the second medicament is potentially more straightforward to seal in the secondary reservoir than a liquid form of the medicament. The device would be used in the same manner as the preferred embodiment with the second medicament being dissolved by the first medicament during dispense.

To minimize diffusion (mixing) of the secondary medicament into the primary medicament during dispense of the primary medicament, the reservoir 22 has an integral flow distributor 23. The role of the flow distribution system is not necessarily to prevent diffusion of the secondary medicament into the container of primary medicament during dispense, to reduce or prevent mixing of the secondary and primary medicaments during dispense, thereby promoting plug flow This flow distributor also ensures efficient expulsion of the second medicament from the system and greatly minimizes residual volume. Preferably the reservoir and flow distributor are manufactured as a single part from materials that are compatible with the secondary medicament, most preferably as a single molded piece. A preferred material would be that typically used to manufacture septa or pistons (bungs) found in multi-dose medicament cartridges, although any material that is compatible with the medicament during long term storage would be equally applicable, for example a material like COP.

The flow distributor 23 is configured and positioned in reservoir 22 such that the secondary medicament fills flow channels that are defined by the shape and location of one or more channels (not shown) inside the reservoir. The shape of the flow channels can be optimized for a plug flow of medicament by varying the dimensions of the flow distributor and/or channels. The cross-sectional area of the annulus formed between the flow distributor and the wall of the reservoir should be kept relatively small. The volume available to store the secondary medicament would equal the internal volume of the reservoir minus the volume of the flow distributor. Therefore if the volume of the flow distributor is marginally smaller than the internal volume of the capsule, a small volume is left which the secondary medicament occupies. Hence the scale of both the capsule and the flow distributor can be large while storing a small volume of medicament. Resultantly for small volumes of secondary medicament (e.g. 50 micro liters) the reservoir can be of an acceptable size for handling, transport, manufacture, filling and assembly.

The cassette of our invention should be designed to operate in conjunction with a multiple use injection device, preferably a pen-type multi-dose injection device, similar to what is illustrated in Fig. 1. The injection device could be a reusable or disposable device. By disposable device it is meant an injection device that is obtained from the manufacturer preloaded with medicament and cannot be reloaded with new medicament after the initial medicament is exhausted. The device may be a fixed dose or a settable dose and preferably a multi-dose device, however, in some cases it may be beneficial to use a single dose, disposable device.

A typical injection device contains a cartridge or other reservoir of primary medication. This cartridge is typically cylindrical in shape and is usually manufactured in glass. The cartridge is sealed at one end with a rubber bung and at the other end by a rubber septum. The injection device is designed to deliver multiple injections. The delivery mechanism is typically powered by a manual action of the user, however, the injection mechanism may also be powered by other means such as a spring, compressed gas or electrical energy. In a preferred embodiment, the delivery mechanism comprises a spindle that engages a piston in the reservoir. In a further embodiment the spindle is a rotatable piston rod comprising two distinct threads.

Exemplary embodiments of the present invention have been described. Those skilled in the art will understand, however, that changes and modifications may be made to these embodiments without departing from the true scope and spirit of the present invention, which is defined by the claims.

List of references
- 3: distal / lower needle
- 4: cassette
- 5: upper / proximal needle
- 6a: top septum / membrane / seal
- 6b: bottom septum/ membrane / seal
- 7: drug delivery device
- 8: cover lock
- 9: connection means/ attachment means
- 10: outer housing
- 11: inner housing
- 13: dose button
- 14: grip surface
- 15: cut-out
- 16: cover
- 17: biasing element / spring
- 18: needle hub
- 19: chassis
- 21: helical path
- 22: reservoir
- 23: flow distributor
- 24: cages
- 26: connector / thread
- 27: priming module
- 28: priming channel
- 32: distal end of device

- 41: indicator
- 50: cartridge holder
- 51: upper hub
- 54: window

## Claims

1. A cassette (4) of medicated modules attachable to a drug delivery device (7), comprising,
an outer housing (10);
an inner housing (11) having a proximal end and a distal end, where the proximal end has an upper hub (51) holding a first double-ended needle cannula (5) and configured for attachment to a drug delivery device (7); and
a primary pack chassis (19) mounted within the outer housing and holding two or more reservoirs (22) each comprising a single dose of a medicament.

2. The cassette of claim 1 where the chassis has two or more cages (24) arranged in a circular pattern that are configured to hold the reservoirs.

3. The cassette of any preceding claim where the cages (24) have an external surface that has a connector (26) that will removably accept a double ended needle cannula mounted in a hub.

4. The cassette of claim 3 where the connector (26) is threaded.

5. The cassette of any preceding claim where the outer housing (10) is configured to be rotated relative to the inner housing (11) causing the chassis (19) to rotate and to expose a cage (24) in a cut-out (15) in the outer housing.

6. The cassette of any preceding claim where a biasing element (17) is positioned between the inner housing (11) and the chassis (19) to assist in rotating the outer housing (10) relative to the inner housing (11).

7. The cassette of any preceding claim where one of the cages (24) contains a priming module (27).

8. The cassette of any preceding claim further comprising a decrementing dose counter.

9. The cassette of any preceding claim where the outer housing (10) has a window (54) for viewing an indicator (41).

10. The cassette of any preceding claim further comprising a cover (16) associated with each cage (24) that is configured to at least partially obscure viewing of the indicator (41) when a removable needle hub is attached to connector (26).

11. The cassette of any preceding claim where an outside surface of the outer housing (10) contains a grip surface (14).

12. The cassette of preceding claims 5-12 where the cut-out (15) is configured to prevent rotation of the outer housing (10) when a needle hub is attached to connector (26).

13. The cassette of any preceding claim further comprising an end of life lockout that prevents further rotation of outer housing (10) and prevents reuse of the cassette.

14. The cassette of any preceding claims where the medicament in the reservoirs (22) comprises one of a GLP-1 and a premix of insulin and a GLP-1.

15. A drug delivery system to deliver two or more medicaments operable through a single dispense interface, comprising,
a primary reservoir (50) of medicament containing at least one drug agent;
a dose button (13) operably connected to the primary reservoir of medicament;
a single dispense interface (3) configured for fluid communication with the primary reservoir; and
the cassette (4) of any of the preceding claims.
